# EUROPÄISCHE PATENTANMELDUNG

(11) **EP 2 862 514 A2**
(43) Veröffentlichungstag der Anmeldung: **22.04.2015**
(21) Anmeldenummer: 14187423.0
(22) Anmeldetag: 02.10.2014
(51) Int. Cl.: A61B 5/083

(54) **Verfahren und Vorrichtung zur Messung der Konzentration von Stickstoffmonoxid in der Atemluft eines Patienten**

(30) Priorität: 17.10.2013 DE 102013221061
(71) Anmelder: ROBERT BOSCH GMBH, 70442 Stuttgart (DE)
(72) Erfinder: Fleischer, Maximilian, 85635 Hoehenkirchen (DE); Pohle, Roland, 85570 Herdweg (DE); Magori, Erhard, 85622 Feldkirchen (DE); von Sicard, Oliver, 80469 Muenchen (DE)

(57) **Zusammenfassung**

Die Erfindung betrifft eine Vorrichtung zur Messung der Konzentration von Stickstoffmonoxid in der Atemluft eines Patienten, umfassend einen Stickstoffdioxid-Sensor (141), der zwischen einer Einlassöffnung (11) und einer Auslassöffnung (16) der Vorrichtung angeordnet ist, und einen Konverter (131) zur Oxidation von Stickstoffmonoxid zu Stickstoffdioxid, der so zwischen der Einlassöffnung (11) und dem Stickstoffdioxid-Sensor (141) angeordnet ist, dass die Vorrichtung in mindestens zwei Zustände geschaltet werden kann, wobei in einen ersten Zustand eine fluidische Verbindung zwischen der Einlassöffnung (11) und dem Stickstoffdioxid-Sensor (141) vorliegt, die nicht durch den Konverter (131) führt und in einen zweiten Zustand eine fluidische Verbindung zwischen der Einlassöffnung (11) und dem Stickstoffdioxid-Sensor (141) vorliegt, die durch den Konverter (131) führt. Hierin erfolgt ein In-Kontakt-bringen einer ersten Atemluftprobe des Patienten mit einem Stickstoffdioxid-Sensor (141, 241), ein Erfassen eines ersten Signals des Stickstoffdioxid-Sensors (141, 241), ein Referenzieren des Stickstoffdioxid-Sensors (141, 241) mittels des ersten Signals, ein Oxidieren von Stickstoffmonoxid in einer zweiten Atemluftprobe des Patienten zu Stickstoffdioxid, ein in Kontakt bringen der zweiten Atemluftprobe mit dem Stickstoffdioxid-Sensor (141, 241), ein Erfassen eines zweiten Signals des Stickstoffdioxid-Sensors (141, 241), und ein Ermitteln der Konzentration von Stickstoffmonoxid in der Atemluft des Patienten aus dem zweiten Signal.

## Beschreibung

Die vorliegende Erfindung betrifft eine Vorrichtung zur Messung der Konzentration von Stickstoffmonoxid in der Atemluft eines Patienten. Weiterhin betrifft die vorliegende Erfindung ein Verfahren zur Messung der Konzentration von Stickstoffmonoxid in der Atemluft eines Patienten, insbesondere unter Verwendung der erfindungsgemäßen Vorrichtung.

### Stand der Technik

Ein nichtinvasives Verfahren zum Erkennen von Krankheiten und metabolischen Fehlfunktionen ist die Messung von Markergasen in der menschlichen Ausatemluft. Solche Messungen können sowohl im Screening als auch in der Differentialdiagnose und in der Therapieoptimierung angewendet werden. Beispielsweise kann eine Messung von Stickstoffmonoxid in der Ausatemluft zur Verlaufskontrolle einer Asthmatherapie, zur Differentialdiagnose von COPD (chronic obstructive pulmonary disease), für die Detektion von Lungentumoren, Tuberkulose und Lungenentzündungen genutzt werden.

Die Messung der Konzentration von Stickstoffmonoxid im Atemgas ist ein wichtiges Mittel zur Optimierung der Behandlung von Asthma. Preiswerte Stickstoffmonoxidsensoren mit der erforderlichen Empfindlichkeit im pbb-Bereich waren bis vor Kurzem nicht am Markt verfügbar. Ein neu entwickelter Stickstoffdioxid-Sensor auf Basis der Suspended Gate Feldeffekttransistor-Technologie (SG-FET), welcher einen Porphinfarbstoff als gassensitive Schicht enthält, entspricht diesen Anforderungen. Allerdings muss dem Sensor ein Konversionsmodul zur Umwandlung des Stickstoffmonoxids (NO) im Atemgas in von dem Sensor detektierbares Stickstoffdioxid (NO₂) vorgeschaltet werden. Zudem benötigt der Sensor vor jeder Messung der Stickstoffdioxidkonzentration kurzzeitig ein stickstoffdioxidfreies Referenzgas, um eine Sensorbasislinie zu erzeugen. Dann werden Änderungen in der Stickstoffdioxidkonzentration detektiert. Die EP 1 384 069 B1 beschreibt eine derartige Vorrichtung zur quantitativen Messung von Stickoxiden in der Ausatemluft. Um den Sensor vor jeder Atemmessung mit Raumluft zu spülen, wird eine Pumpe benötigt, die Umgebungsluft in die Messkammer der Vorrichtung pumpt. Da die Umgebungsluft abhängig von den Umweltbedingungen eine Stickstoffdioxidkonzentration von bis zu mehreren 10 ppb enthalten kann, muss diese Luft zunächst durch einen Aktivkohlefilter gepumpt werden. Dabei wird der Filter verbraucht. Die so zubereitete Referenzluft unterscheidet sich allerdings hinsichtlich ihres Feuchtigkeitsgehalts und ihrer Temperatur deutlich von der menschlichen Atemluft, die eine Temperatur von ca. 35°C und eine relative Feuchte von 100 % aufweist. Daher wird das Sensorsignal nicht nur durch eine sich ändernde Stickstoffdioxidkonzentration beeinflusst.

### Offenbarung der Erfindung

Die erfindungsgemäße Vorrichtung zur Messung der Konzentration von Stickstoffmonoxid in der Atemluft eines Patienten umfasst einen Stickstoffdioxid-Sensor, der zwischen einer Einlassöffnung und einer Auslassöffnung der Vorrichtung angeordnet ist und einen Konverter zur Oxidation von Stickstoffmonoxid zu Stickstoffdioxid, der so zwischen der Einlassöffnung dem Stickstoffdioxid-Sensor angeordnet ist, dass die Vorrichtung in mindestens zwei Zustände geschaltet werden kann, wobei in einem ersten Zustand eine fluidische Verbindung zwischen der Einlassöffnung und dem Stickstoffdioxid-Sensor vorliegt, die nicht durch den Konverter führt und einem zweiten Zustand der fluidischen Verbindung zwischen der Einlassöffnung und dem Stickstoffdioxid-Sensor vorliegt, die durch den Konverter führt. In dem ersten Zustand liegt vorzugsweise keine fluidische Verbindung zwischen der Einlassöffnung und dem Stickstoffdioxid-Sensor vor, die nicht durch den Konverter führt. In dem zweiten Zustand liegt vorzugsweise keine fluidische Verbindung zwischen der Einlassöffnung und dem Stickstoffdioxid-Sensor vor, die durch den Konverter führt. Diese Vorrichtung ermöglicht die Messung der Konzentration von Stickstoffmonoxid in der Atemluft eines Patienten mittels des erfindungsgemäßen Verfahrens. Dieses umfasst das in Kontakt bringen einer ersten Atemluftprobe des Patienten mit einem Stickstoffdioxid-Sensor, das Erfassen eines ersten Signals des Stickstoffdioxid-Sensors, das Referenzieren des Stickstoffdioxid-Sensors mittels des ersten Signals, das Oxidieren von Stickstoffmonoxid in einer zweiten Atemluftprobe des Patienten zu Stickstoffdioxid, das in Kontakt bringen der zweiten Atemluftprobe mit dem Stickstoffdioxid-Sensor, das Erfassen eines zweiten Signals des Stickstoffdioxid-Sensors und das Ermitteln der Konzentration von Stickstoffmonoxid in der Atemluft des Patienten aus dem zweiten Signal. Unter einer Atemluftprobe wird erfindungsgemäß eine Atemluftmenge oder ein Teil einer Atemluftmenge des Patienten verstanden, die bzw. der in dem erfindungsgemäßen Verfahren untersucht wird. Es ist möglich, dass der Patient die erste Atemluftprobe und die zweite Atemluftprobe bereitstellt, ohne dabei seinen Ausatemvorgang zu unterbrechen. Die menschliche Ausatemluft enthält unabhängig von den Umgebungsbedingungen kein Stickstoffdioxid. Die erfindungsgemäße Vorrichtung benötigt daher keinen Aktivkohlefilter mit begrenzter Lebensdauer und keine Pumpe zum Spülen des Stickstoffdioxid-Sensors. Zudem wird in dem erfindungsgemäßen Verfahren als stickstoffdioxidfreies Referenzgas für den Stickstoffdioxid-Sensor menschliche Atemluft, also ein Gas mit den gleichen Restgasen und mit denselben Eigenschaften, insbesondere Temperatur und Feuchte, zur Verfügung gestellt, die auch die zu untersuchende Atemluftprobe während der Stickstoffdioxidmessung zur Ermittlung der Konzentration von Stickstoffmonoxid in der Atemluft aufweisen.

In einer bevorzugten Ausführungsform der erfindungsgemäßen Vorrichtung ist zwischen der Einlassöffnung und dem Stickstoffdioxid-Sensor ein Wechselelement angeordnet, das den Konverter und ein Verbindungselement, welches keine Oxidation von Stickstoffmonoxid zu Stickstoffdioxid vornehmen kann, umfasst, wobei wahlweise der Konverter oder das Verbindungselement in die fluidische Verbindung zwischen der Einlassöffnung und dem Stickstoffdioxid-Sensor hineinbewegbar ist. Dies ermöglicht es in einer bevorzugten Ausführungsform des erfindungsgemäßen Verfahrens nach dem Erfassen des ersten Signals den Konverter zur Oxidation von Stickstoffmonoxid zu Stickstoffdioxid in die fluidische Verbindung zwischen der Einlassöffnung zum Einlass von Atemluft des Patienten und den Stickstoffdioxid-Sensor zu bewegen und den Konverter nach dem Erfassen des zweiten Signals aus der fluidischen Verbindung herauszubewegen. Hierzu wird besonders bevorzugt das in der fluidischen Verbindung angeordnete Wechselelement genutzt, das den Konverter und das Verbindungselement, welches keine Oxidation von Stickstoffmonoxid zu Stickstoffdioxid vornehmen kann, umfasst, sodass beim Herausbewegen des Konverters aus der fluidischen Verbindung das Verbindungselement in die fluidische Verbindung hineinbewegt wird.

In einer anderen bevorzugten Ausführungsform der erfindungsgemäßen Vorrichtung sind zwischen der Einlassöffnung und dem Stickstoffdioxid-Sensor zwei fluidische Verbindungen angeordnet, die, insbesondere unabhängig voneinander, jeweils mit einem Ventil verschließbar sind, wobei der Konverter in einer dieser Verbindungen angeordnet ist und in der anderen Verbindung kein Element angeordnet ist, das eingerichtet ist, um eine Oxidation von Stickstoffmonoxid zu Stickstoffdioxid vorzunehmen. Dies ermöglicht es in einer bevorzugten Ausführungsform des erfindungsgemäßen Verfahrens, nach dem Erfassen des ersten Signals eine erste fluidische Verbindung zwischen einer Einlassöffnung zum Einlassen von Atemluft des Patienten und dem Stickstoffdioxid-Sensor zu verschließen und eine zweite fluidische Verbindung, in der ein Konverter zur Oxidation von Stickstoffmonoxid zu Stickstoffdioxid angeordnet ist, zwischen der Einlassöffnung und dem Stickstoffdioxid-Sensor zu öffnen und nach dem Erfassen des zweiten Signals die zweite fluidische Verbindung zu schließen und die erste fluidische Verbindung zu öffnen. Dabei ist es besonders bevorzugt, dass das Öffnen und Schließen der ersten fluidischen Verbindung mit der zweiten fluidischen Verbindung mittels Ventilen erfolgt. Ein Ventil ist in einer ganz besonders bevorzugten Ausführungsform des Verfahrens stromaufwärts des Konverters angeordnet. In einer anderen ganz besonders bevorzugten Ausführungsform des Verfahrens ist ein Ventil stromabwärts des Konverters angeordnet. Hierbei kann es sich insbesondere um ein Dreiwegeventil handeln, das sowohl das Öffnen und Schließen der ersten fluidischen Verbindung als auch das Öffnen und Schließen der zweiten fluidischen Verbindung ermöglicht.

In noch einer anderen bevorzugten Ausführungsform umfasst die erfindungsgemäße Vorrichtung einen Strömungsteiler, der eingerichtet ist, um einen ersten Teil einer Fluidströmung zwischen der Einlassöffnung und der Auslassöffnung durch den Konverter zu leiten und einen zweiten Teil der Fluidströmung nicht durch den Konverter zu leiten, wobei das Verhältnis zwischen dem ersten Teil und dem zweiten Teil durch einen Benutzer änderbar ist. Dies ermöglicht es in einer bevorzugten Ausführungsform des erfindungsgemäßen Verfahrens, vor Erfassen des ersten Signals des Stickstoffdioxid-Sensors einen hohen Atemluftfluss durch den Konverter und einen geringen Atemluftfluss an dem Konverter vorbei zu erzeugen und vor Erfassen des zweiten Signals des Stickstoffdioxid-Sensors einen geringen Atemluftfluss durch den Konverter und einen hohen Atemluftfluss an dem Konverter vorbei zu erzeugen.

Es ist bevorzugt, dass der Stickstoffdioxid-Sensor in einer Messkammer angeordnet ist und dass in einer fluidischen Verbindung der Messkammer mit der Auslassöffnung ein Ventil angeordnet ist. Dies ermöglicht es, in einer bevorzugten Ausführungsform des erfindungsgemäßen Verfahrens während des Erfassens des ersten Signals und während des Erfassens des zweiten Signals eine fluidische Verbindung zwischen der Messkammer und einer Einlassöffnung zum Einlassen von Atemluft des Patienten in die Messkammer zu schließen und eine fluidische Verbindung zwischen der Messkammer und einer Auslassöffnung zum Auslassen von Atemluft des Patienten aus der Messkammer zu schließen. Dadurch kann die Messung des ersten Signals und die Messung des zweiten Signals nicht durch von außen an den Stickstoffdioxid-Sensor heranströmende Luft verfälscht werden.

### Kurze Beschreibung der Zeichnungen

Ausführungsbeispiele der Erfindung sind in den Zeichnungen schematisch dargestellt und in der nachfolgenden Beschreibung näher erläutert.
- Fig. 1: ist eine schematische Darstellung einer Vorrichtung gemäß einer Ausführungsform der Erfindung.
- Fig. 2: ist eine schematische Darstellung einer Vorrichtung gemäß einer anderen Ausführungsform der Erfindung.
- Fig. 3: zeigt den zeitlichen Verlauf eines ersten Stickstoffdioxid-Sensorsignals in einem Verfahren gemäß einer Ausführungsform der Erfindung.
- Fig. 4: zeigt den zeitlichen Verlauf eines zweiten Stickstoffdioxid-Sensorsignals in einem Verfahren gemäß einer Ausführungsform der Erfindung.

### Ausführungsbeispiele der Erfindung

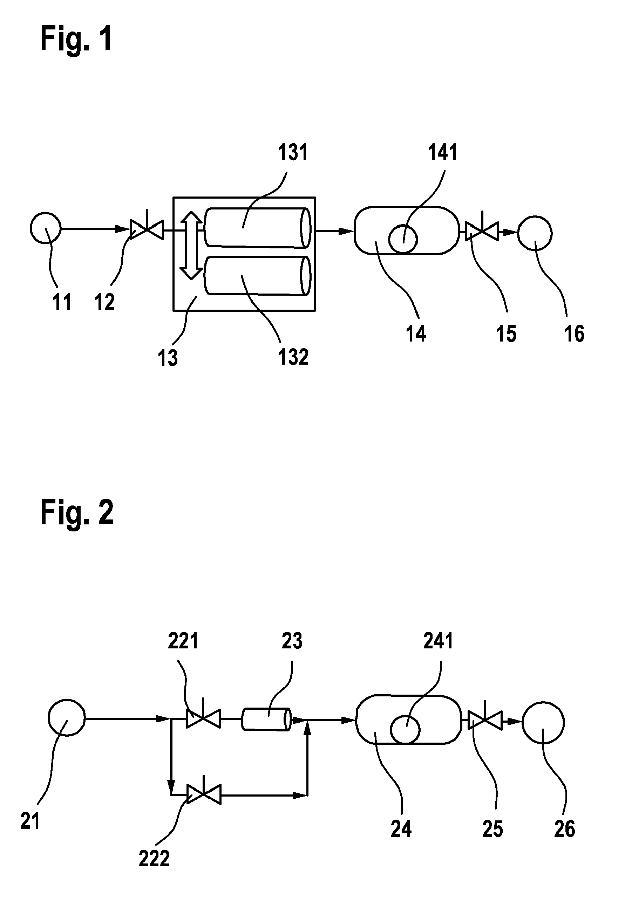

Eine erste Ausführungsform einer erfindungsgemäßen Vorrichtung zur Messung der Konzentration von Stickstoffmonoxid in der Atemluft eines Patienten ist schematisch in Fig. 1 gezeigt. Eine Einlassöffnung 11 ist fluidisch mit einem Einlassventil 12 verbunden. Das Einlassventil 12 ist fluidisch mit einer Messkammer 14 verbunden. In der fluidischen Verbindung zwischen dem Einlassventil 12 und der Messkammer 14 ist ein Wechselelement 13 angeordnet. Dieses enthält einen Konverter 131 und ein Verbindungselement 132. Der Konverter enthält Kaliumpermanganat auf Zeolith als Trägermaterial. Das Verbindungselement 132 entspricht in seinen Abmessungen dem Konverter 131, ist jedoch leer. Das Wechselelement 13 ist ausgebildet, dass jeweils entweder der Konverter 131 oder das Verbindungselement 132 in die fluidische Verbindung zwischen dem Einlassventil 12 und der Messkammer 14 hineinbewegbar ist. Die Messkammer 14 enthält einen SG-FET-Stickstoffdioxid-Sensor 141. Die Messkammer 14 ist fluidisch mit einer Auslassöffnung 16 verbunden, wobei diese fluidische Verbindung durch ein Auslassventil 15 führt.

In einer ersten Ausführungsform des erfindungsgemäßen Verfahrens bläst ein Patient zunächst eine erste Atemgasportion von 100 bis 500 ml durch die Einlassöffnung 11, das Einlassventil 12 und das Verbindungselement 132 in die Messkammer 14. Dann werden das Einlassventil 12 und das Auslassventil 15 geschlossen und der Stickstoffdioxid-Sensor 141 erfasst ein erstes Signal. Anschließend werden die beiden Ventile 12, 15 wieder geöffnet und der Konverter 131 und das Verbindungselement 132 tauschen in dem Wechselelement 13 ihre Position. Nun bläst der Patient eine zweite Atemgasportion durch die Einlassöffnung 11, das Einlassventil 12 und den Konverter 131 in die Messkammer 14. Diese zweite Atemgasportion wird im Konverter 131 mit dem Kaliumpermanganat in Kontakt gebracht. Hierdurch wird in dem Atemgas enthaltenes Stickstoffmonoxid zu Stickstoffdioxid oxidiert:

3 NO + 2 KMnO₄ + H₂O → 3 NO₂ + 2 MnO₂ + 2 KOH

Die zweite Atemgasportion verdrängt die erste Atemgasportion aus der Messkammer 14, welche durch das Auslassventil 15 und die Auslassöffnung 16 die Vorrichtung verlässt. Anschließend werden die Ventile 12, 15 geschlossen und der Sensor 141 erfasst ein zweiten Stickstoffdioxidsignal. Danach werden die Ventile 12, 15 wieder geöffnet. Aus der Differenz zwischen dem ersten Signal und dem zweiten Signal des Stickstoffdioxid-Sensors 141 wird der Beitrag des durch Oxidation von Stickstoffmonoxid zu Stickstoffdioxid erhaltenen Stickstoffdioxids am gemessenen Stickstoffdioxid ermittelt. Die Stoffmenge dieses Stickstoffdioxids entspricht der in der zweiten Atemgasportion enthaltenen Stoffmenge an Stickstoffmonoxid.

Eine Vorrichtung gemäß einer zweiten Ausführungsform der Erfindung ist schematisch in der Fig. 2 gezeigt. Eine Einlassöffnung 21 ist fluidisch mit einer Messkammer 24 verbunden. In dieser Verbindung sind ein erstes Einlassventil 221 und ein KMnO₄/Zeolith-Konverter 23 angeordnet. Zwischen der Einlassöffnung 21 und dem ersten Einlassventil 221 zweigt ein Weg der fluidischen Verbindung ab, welcher durch ein zweites Einlassventil 222 geführt wird und zwischen dem Konverter 23 und der Messkammer 24 endet. Die Messkammer 24 enthält einen SG-FET-Stickstoffdioxid-Sensor241. Die Messkammer 24 ist mit einer Auslassöffnung 26 fluidisch verbunden, wobei in dieser fluidischen Verbindung ein Auslassventil 25 angeordnet ist.

Zunächst ist das erste Einlassventil 221 geschlossen und das zweite Einlassventil 222 und das Auslassventil 25 sind geöffnet. Ein Patient kann nun in einer zweiten Ausführungsform des erfindungsgemäßen Verfahrens eine erste Atemgasportion durch die Einlassöffnung 21 und das zweite Einlassventil 222 in die Messkammer 24 einblasen. Nun werden das zweite Einlassventil 222 und das Auslassventil 25 geschlossen und der Stickstoffdioxid-Sensor 241 erfasst ein erstes Signal. Dann werden das Auslassventil 25 und das erste Einlassventil 221 geöffnet. Anschließend bläst der Patient eine zweite Atemgasportion durch die Einlassöffnung 21, das erste Einlassventil 221 und den Konverter 23 in die Messkammer 24 ein und verdrängt dabei die erste Atemgasportion aus der Messkammer 24 durch das Auslassventil 25 und der Auslassöffnung 26 hinaus. Anschließend werden die beiden geöffneten Ventile 221, 25 geschlossen und der Stickstoffdioxid-Sensor 241 erfasst ein zweites Signal. Schließlich werden das zweite Einlassventil 222 und das Auslassventil 25 wieder geöffnet. Die Ermittlung des Stickstoffmonoxidgehalts in der zweiten Atemgasportion des Patienten aus dem ersten und dem zweiten Sensorsignal erfolgt in derselben Weise wie in der ersten Ausführungsform des erfindungsgemäßen Verfahrens.

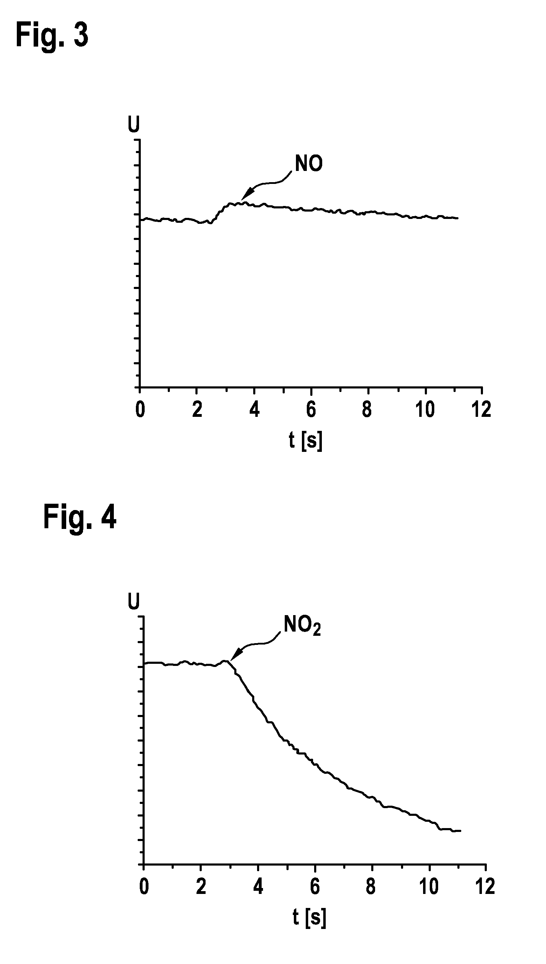

Fig. 3 zeigt den Verlauf eines Sensorspannungssignals U mit der Zeit t, nachdem an einem Punkt NO eine erste Atemgasportion in eine Messkammer 14, 24 der ersten oder der zweiten Ausführungsform der erfindungsgemäßen Vorrichtung eingeblasen wurde. Diese Atemgasportion enthält 30 ppb Stickstoffmonoxid, was aber zu keiner relevanten Änderung des Spannungssignals U führt. Fig. 4 zeigt den zeitlichen Verlauf des Sensorspannungssignals U nach Einblasen der zweiten Atemgasportion in die Messkammer 14, 24 einer Vorrichtung gemäß der ersten und der zweiten Ausführungsform der Erfindung. Nachdem am Punkt NO₂ 30 ppb Stickstoffdioxid, das durch Oxidation von 30 ppb Stickstoffmonoxid im Konverter 131, 23 erzeugt wurde, mit dem Stickstoffdioxid-Sensor 141, 241 in Kontakt gelangt, ist ein deutlicher Spannungsabfall zu beobachten. Durch Subtraktion der beiden Stickstoffdioxid-Sensorsignale aus den Fig. 3 und 4 kann der Stickstoffdioxidstoffmengenanteil der zweiten Atemgasportion ermittelt werden, welcher deren Stickstoffmonoxidstoffmengenanteil entspricht.

## Patentansprüche

1. Vorrichtung zur Messung der Konzentration von Stickstoffmonoxid in der Atemluft eines Patienten, umfassend einen Stickstoffdioxid-Sensor (141, 241), der zwischen einer Einlassöffnung (11, 21) und einer Auslassöffnung (16, 26) der Vorrichtung angeordnet ist, und einen Konverter (131, 23) zur Oxidation von Stickstoffmonoxid zu Stickstoffdioxid, der so zwischen der Ein-lassöffnung (11, 21) und dem Stickstoffdioxid-Sensor (141, 241) angeordnet ist, dass die Vorrichtung in mindestens zwei Zustände geschaltet werden kann, wobei in einem ersten Zustand eine fluidische Verbindung zwischen der Einlassöffnung (11, 21) und dem Stickstoffdioxid-Sensor (141, 241) vorliegt, die nicht durch den Konverter (131, 23) führt und in einem zweiten Zustand eine fluidische Verbindung zwischen der Einlassöffnung (11, 21) und dem Stickstoffdioxid-Sensor (141, 241) vorliegt, die durch den Konverter (131, 23) führt.

2. Vorrichtung nach Anspruch 1, **dadurch gekennzeichnet, dass** in dem ersten Zustand keine fluidische Verbindung zwischen der Einlassöffnung (11, 21) und dem Stickstoffdioxid-Sensor (141, 241) vorliegt, die nicht durch den Konverter (131, 23) führt und in dem zweiten Zustand keine fluidische Verbindung zwischen der Einlassöffnung (11, 21) und dem Stickstoffdioxid-Sensor (141, 241) vorliegt, die durch den Konverter (131, 23) führt.

3. Vorrichtung nach Anspruch 1 oder 2, **dadurch gekennzeichnet, dass** zwischen der Einlassöffnung (11) und dem Stickstoffdioxid-Sensor (141) ein Wechselelement (13) angeordnet ist, das den Konverter (131) und ein Verbindungselement (132), welches keine Oxidation von Stickstoffmonoxid zu Stickstoffdioxid vornehmen kann, umfasst, wobei wahlweise der Konverter (131) oder das Verbindungselement (132) in die fluidische Verbindung zwischen der Einlassöffnung (11) und dem Stickstoffdioxid-Sensor (141) hineinbewegbar ist.

4. Vorrichtung nach Anspruch 1 oder 2, **dadurch gekennzeichnet, dass** zwischen der Einlassöffnung (21) und dem Stickstoffdioxid-Sensor (241) zwei fluidische Verbindungen angeordnet sind, die jeweils mit einem Ventil (221, 222) verschließbar sind, wobei der Konverter (23) in einer dieser Verbindungen angeordnet ist und in der anderen Verbindung kein Element angeordnet ist, das eingerichtet ist, um eine Oxidation von Stickstoffmonoxid zu Stickstoffdioxid vorzunehmen.

5. Vorrichtung nach Anspruch 1, **dadurch gekennzeichnet, dass** sie einen Strömungsteiler umfasst, der eingerichtet ist, um einen ersten Teil einer Fluidströmung zwischen der Einlassöffnung und der Auslassöffnung durch den Konverter zu leiten und einen zweiten Teil der Fluidströmung nicht durch den Konverter zu leiten, wobei das Verhältnis zwischen dem ersten Teil und dem zweiten Teil durch einen Benutzer änderbar ist.

6. Vorrichtung nach einem der Ansprüche 1 bis 5, **dadurch gekennzeichnet, dass** der Stickstoffdioxid-Sensor (141, 241) in einer Messkammer (14, 24) angeordnet ist, und dass in einer fluidischen Verbindung der Messkammer (14, 24) mit der Auslassöffnung (16, 26) ein Ventil (15, 16) angeordnet ist.

7. Verfahren zum zur Messung der Konzentration von Stickstoffmonoxid in der Atemluft eines Patienten, umfassend
- in Kontakt bringen einer ersten Atemluftprobe des Patienten mit einem Stickstoffdioxid-Sensor (141, 241),
- Erfassen eines ersten Signals des Stickstoffdioxid-Sensors (141, 241),
- Referenzieren des Stickstoffdioxid-Sensors (141, 241) mittels des ersten Signals,
- Oxidieren von Stickstoffmonoxid in einer zweiten Atemluftprobe des Patienten zu Stickstoffdioxid,
- In-Kontakt-bringen der zweiten Atemluftprobe mit dem Stickstoffdioxid-Sensor (141, 241),
- Erfassen eines zweiten Signals des Stickstoffdioxid-Sensors (141, 241), und
- Ermitteln der Konzentration von Stickstoffmonoxid in der Atemluft des Patienten aus dem zweiten Signal.

8. Verfahren nach Anspruch 7, **dadurch gekennzeichnet, dass** nach dem Erfassen des ersten Signals ein Konverter (131) zur Oxidation von Stickstoffmonoxid zu Stickstoffdioxid, in eine fluidische Verbindung zwischen einer Einlassöffnung (11) zum Einlassen von Atemluft des Patienten und den Stickstoffdioxid-Sensor (141) bewegt wird, und dass der Konverter (131) nach dem Erfassen des zweiten Signals aus der fluidischen Verbindung herausbewegt wird.

9. Verfahren nach Anspruch 8, **dadurch gekennzeichnet, dass** in der fluidischen Verbindung ein Wechselelement (13) angeordnet ist, das den Konverter (131) und ein Verbindungselement (132), welches keine Oxidation von Stickstoffmonoxid zu Stickstoffdioxid vornehmen kann, umfasst, und dass beim Herausbewegen des Konverters (131) aus der fluidischen Verbindung das Verbindungselement (132) in die fluidische Verbindung hineinbewegt wird.

10. Verfahren nach Anspruch 7, **dadurch gekennzeichnet, dass** nach dem Erfassen des ersten Signals eine erste fluidische Verbindung zwischen einer Einlassöffnung (21) zum Einlassen von Atemluft des Patienten und dem Stickstoffdioxid-Sensor (241) verschlossen wird, und eine zweite fluidische Verbindung, in der ein Konverter (23) zur Oxidation von Stickstoffmonoxid zu Stickstoffdioxid angeordnet ist, zwischen der Einlassöffnung (21) und dem Stickstoffdioxid-Sensor (241) geöffnet wird, und dass nach dem Erfassen des zweiten Signals die zweite fluidische Verbindung geschlossen wird und die erste fluidische Verbindung geöffnet wird.

11. Verfahren nach Anspruch 10, **dadurch gekennzeichnet, dass** das Öffnen und Schließen der ersten fluidischen Verbindung und der zweiten fluidischen Verbindung mittel Ventilen (221, 222) erfolgt.

12. Verfahren nach einem der Ansprüche 7 bis 11, **dadurch gekennzeichnet, dass** der Stickstoffdioxid-Sensor (141, 241) in einer Messkammer (14, 24) angeordnet ist, und dass während des Erfassens des ersten Signals und während des Erfassens des zweiten Signals eine fluidische Verbindung zwischen der Messkammer (14, 24) und einer Einlassöffnung (11, 21) zum Einlassen von Atemluft des Patienten in die Messkammer (14, 24) geschlossen wird und eine fluidische Verbindung zwischen der Messkammer (14, 24) und einer Auslassöffnung (16, 26) zum Auslassen von Atemluft des Patienten aus der Messkammer (14, 24) geschlossen wird.
